# EUROPÄISCHE PATENTSCHRIFT

(11) **EP 0 964 863 B1**
(45) Veröffentlichungstag und Bekanntmachung des Hinweises auf die Patenterteilung: **21.05.2003**
(21) Anmeldenummer: 98909397.6
(22) Anmeldetag: 06.02.1998
(51) Int. Cl.: C07D 413/14, A61K 31/445, C07D 491/056

(54) **OXAZOLIDINONE ALS 5-HT2A-ANTAGONISTEN**
OXAZOLIDINES AS 5-HT2A-ANTAGONISTS
OXAZOLIDINONES S'UTILISANT COMME 5-HT2A-ANTAGONISTES

(30) Priorität: 26.02.1997 DE 19707628
(43) Veröffentlichungstag der Anmeldung: 22.12.1999
(73) Patentinhaber: MERCK PATENT GmbH, 64271 Darmstadt (DE)
(72) Erfinder: BÖTTCHER, Henning, D-64287 Darmstadt (DE); PRÜCHER, Helmut, D-64646 Heppenheim (DE); GREINER, Helmut, D-64331 Weiterstadt (DE); BARTOSZYK, Gerd, D-64331 Weiterstadt (DE); SEYFRIED, Christoph, D-64342 Seeheim (DE)
(86) Internationale Anmeldenummer: EP9800637
(87) Internationale Veröffentlichungsnummer: WO98038189

(56) Entgegenhaltungen:
- EP-A- 0 459 256
- EP-A- 0 635 505
- EP-A- 0 722 942
- WO-A-92/00070
- DE-A- 3 723 797

## Beschreibung

Die Erfindung betrifft Verbindungen der Formel I 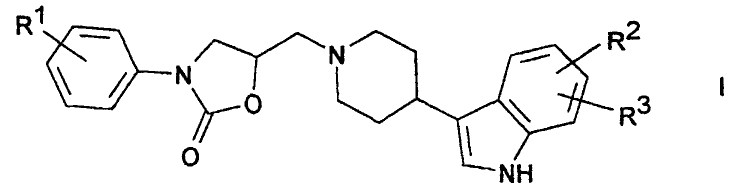 worin
- R¹: H, CN, Hal oder OA,
- R², R³: jeweils unabhängig voneinander H, CN, Hal oder OA,
- R² und R³: zusammen auch Methylendioxy,
- A: H, CF₃ oder Alkyl mit 1-6 C-Atomen und
- Hal: F, Cl, Br, I
bedeuten,
sowie deren Salze.

5-[(2-Oxo-benzimidazolin-1-yl)-piperidino-methyl]-oxazolidin-2-one mit Wirkungen auf das Zentralnervensystem sind z.B. aus der EP 0 443 197 bekannt.
Durch Indolalkylreste N-alkylierte Indolpiperidine sind z.B. in der EP 0 683 166 beschrieben.
3-Phenyl-5-[(4-R-X-piperidino)-alkyl]-oxazolidin-2-on-Derivate, worin R Phenyl und X -O-, -S-, -SO- oder -SO₂- bedeutet, mit Wirkungen auf das Zentralnervensystem sind z.B. aus der EP 0 635 505 bekannt.
Indolpiperidin-Derivate mit tricyclischem Rest und Wirkungen auf das Zentralnervensystem sind z.B. in der EP 0 722 942 beschrieben.
4-Aryl-1-(indan-, dihydrobenzofuran- oder dihydrobenzothiophen-methyl)-piperidin-Derivate mit Einfluß auf die serotoninerge und dopaminerge Transmission als auch mit 5-HT-Reuptake-hemmender Wirkung, sind z.B. in der WO 95/33721 beschrieben.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit besonders wertvolle pharmakologische Eigenschaften besitzen, da sie Wirkungen auf das Zentralnervensystem, insbesondere Dopamin-antagonistische und 5-HT-reuptake hemmende Wirkungen, aufweisen, indem sie sowohl die serotoninerge als auch die dopaminerge Transmission beeinflussen. Insbesondere weisen sie Affinitäten zu den 5-HT_{1A}- und / oder 5-HT_{2A}-Rezeptoren auf.

Die Verbindungen der Formel I hemmen die Bindung von tritiierten Serotoninrezeptorliganden an hippocampale Rezeptoren (Cossery et al., European J. Pharmacol. 140 (1987), 143-155) und hemmen die synaptosomale Serotoninwiederaufnahme (Sherman et al., Life Sci. 23 (1978), 1863 - 1870). Insbesondere binden sie an 5-HT_{2A}-und D₂-Rezeptoren. Außerdem treten Veränderungen der DOPA-Akkumulation im Striatum und der 5-HTP-Akkumulation in N.raphesauf (Seyfried et al., European J. Pharmacol. 160 (1989), 31 - 41). Die 5-HT_{1A}- antagonistische Wirkung wird in vitro z. b. durch Hemmung der 8-OH-DPAT-verursachte Aufhebung der elektrisch induzierten Kontraktion des Meerschweinchenileums nachgewiesen (Fozard und Kilbinger, Br. J. Pharmacol. 86 (1985) 601P). Ex-vivo dient zum Nachweis der 5-HT_{1A}-antagonistischen Wirkung die Hemmung der durch 8-OH-DPAT verminderten 5-HTP-Akkumulation (Seyfried et al., European J. Pharmacol. 160 (1989), 31-41).
Zum ex vivo Nachweis der Serotoninwiederaufnahmehemmung wird die synaptosomale Aufnahmehemmung (Wong et al. Neuropsychopharmacol. 8 (1993), 23-33) und der p-Chloramphetaminantagonismus (Fuller et al., J. Pharmacol. Exp. Ther. 212 (1980), 115-119) herangezogen.
Im übrigen können die pharmakologischen Tests analog der Methoden durchgeführt werden, wie sie in der WO 95/33721 beschrieben sind.

Die Verbindungen der Formel I eignen sich daher sowohl in der Veterinärals auch in der Humanmedizin zur Behandlung von Funktionsstörungen des Zentralnervensystems. Sie können zur Prophylaxe und zur Bekämpfung von Folgen cerebraler Infarktgeschehen (apoplexia cerebri) wie Schlaganfall und cerebraler Ischämien, sowie zur Behandlung extrapyramidal-motorischer Nebenwirkungen von Neuroleptika sowie des Morbus Parkinson verwendet werden. Insbesondere sind sie jedoch geeignet als Arzneimittelwirkstoffe für Anxiolytika, Antidepressiva, Antipsychotika und/oder zur Behandlung von Zwangsverhalten (obsessive-compulsive disorder, OCD), Angstzuständen, Panikattacken, Depressionen, Psychosen, Schizophrenie, wahnhaften Zwangsvorstellungen, der Alzheimer Krankheit, Migräne, Anorexie, Schlafstörungen, tardiver Dyskinesien, Lernstörungen, altersabhängiger Erinnerungsstörungen, Eßstörungen wie Bulimie, Drogenmißbrauch und/oder Sexualfunktionsstörungen.

Die Verbindungen der Formel I und ihre physiologisch unbedenklichen Säureadditionssalze können daher als Arzneimittelwirkstoffe für Anxiolytika, Antidepressiva, Antipsychotika, Neuroleptika und/oder Antihypertonika sowie zur positiven Beeinflussung von Zwangsverhalten, Eßstörungen wie Bulimie, tardiver Dyskinesien, Lernstörungen und altersabhängiger Erinnerungsstörungen verwendet werden.
Ferner können sie als Zwischenprodukte zur Herstellung weiterer Arzneimittelwirkstoffe eingesetzt werden.

Gegenstand der Erfindung sind somit die Verbindungen der Formel I sowie ihre physiologisch unbedenklichen Säureadditionssalze.

Gegenstand der Erfindung sind dementsprechend die Verbindungen der Formel I sowie ein Verfahren zur Herstellung von Verbindungen der Formel I, dadurch gekennzeichnet, daß man
a) eine Verbindung der Formel II 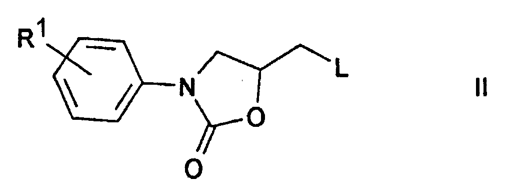 worin R¹ die in Anspruch 1 angegebene Bedeutung hat und L Cl, Br, I oder eine freie oder reaktionsfähig funktionell abgewandelte OH-Gruppe bedeutet,
   mit einer Verbindung der Formel III 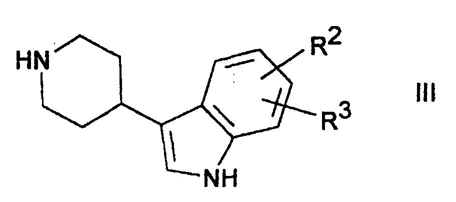 worin R² und R³ die in Anspruch 1 gegebenen Bedeutungen haben,
   umsetzt,
   oder
b) eine Verbindung der Formel IV 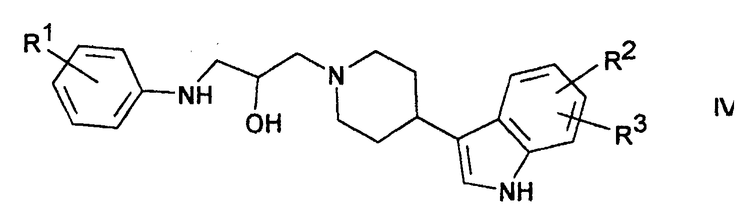 worin R¹, R² und R³ die in Anspruch 1 angegebenen Bedeutungen haben,
   mit einer Verbindung der Formel V 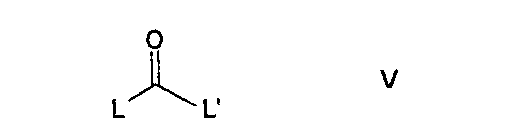 worin L und L' jeweils unabhängig voneinander Cl, Br, I oder eine freie oder reaktionsfähig funktionell abgewandelte OH-Gruppe bedeutet,
   umsetzt,
   oder
c) eine Verbindung der Formel VI 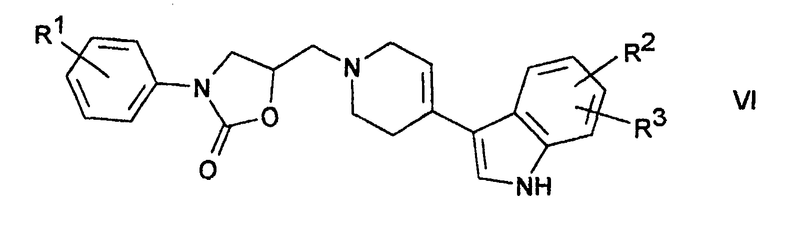
worin R¹, R² und R³ die in Anspruch 1 angegebenen Bedeutungen haben,
hydriert,
und/oder daß man eine basische Verbindung der Formel I durch Behandeln mit einer Säure in eines ihrer Salze überführt.

Vor- und nachstehend haben die Reste R¹, R², R³ und L die bei den Formeln I, II, III, IV und V angegebenen Bedeutungen, sofern nicht ausdrücklich etwas anderes angegeben ist.

Gegenstand der Erfindung sind ebenfalls Arzneimittel der Formel I und ihre physiologisch unbedenklichen Salze mit 5-HT_{1A}- , 5-HT_{2A}- antagonistischer und 5-HT-reuptake-hemmender Wirkung.

Gegenstand der Erfindung sind die Verbindungen der Formel I gemäß Anspruch 1 sowie deren Enantiomere und deren Salze.

Für alle Reste, die mehrfach auftreten, wie z. B. A, gilt, daß deren Bedeutungen unabhängig voneinander sind.

Alkyl hat 1 bis 10, vorzugsweise 1, 2, 3, 4, 5 oder 6 C-Atome. Alkyl bedeutet daher insbesondere z.B. Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, ferner auch Fluormethyl, Difluormethyl, Trifluormethyl, 1,1,1-Trichlorethyl oder Pentafluorethyl.

A-O- bedeutet Hydroxy oder Alkoxy, insbesondere z.B. Methoxy, Ethoxy, Propoxy oder Butyloxy.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart), beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

L bedeutet in den Verbindungen der Formel II, bzw. L und L' in den Verbindungen der Formel V, jeweils unabhängig voneinander Cl, Br, I oder eine freie oder reaktionsfähige veresterte OH-Gruppe.

Falls L eine reaktionsfähige veresterte OH-Gruppe bedeutet, so ist diese vorzugsweise Trichlormethoxy, Alkoxy, wie z.B. Methoxy, Ethoxy, Propoxy oder Butoxy, weiter Phenoxy, Alkylsulfonyloxy mit 1-6 C-Atomen (bevorzugt Methylsulfonyloxy) oder Arylsulfonyloxy mit 6-10 C-Atomen (bevorzugt Phenyl- oder p-Tolylsulfonyloxy, ferner auch 2-Naphthalinsulfonyloxy). Insbesondere in den Verbindungen der Formel V bedeutet L Cl, 1-Imidazolyl, Ethoxy, Trichlormethoxy, Phenoxy oder Nitrophenoxy.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.
Andererseits ist es möglich, die Reaktion stufenweise durchzuführen.

Die Verbindungen der Formel I können vorzugsweise erhalten werden, indem man Verbindungen der Formel II mit Verbindungen der Formel III umsetzt.

Die Ausgangsstoffe der Formeln II und III sind teilweise bekannt. Sofern sie nicht bekannt sind, können sie nach an sich bekannten Methoden hergestellt werden.

Primäre Alkohole der Formel II sind z.B. durch Reduktion der entsprechenden Carbonsäuren oder ihrer Ester erhältlich. Behandlung mit Thionylchlorid, Bromwasserstoff, Phosphortribromid oder ähnlichen Halogenverbindungen liefert die entsprechenden Halogenide der Formel II.

3-(4-Piperidyl)-indole der Formel III können z.B. durch Umsetzung von 4-Piperidonen mit Indol oder entsprechenden durch R² und/oder R³ substituierten Derivaten mit anschließender Säurebehandlung und Hydrierung hergestellt werden.

Die Umsetzung der Verbindungen der Formel II mit Verbindungen der Formel III erfolgt in der Regel in einem inerten Lösungsmittel, in Gegenwart eines säurebindenden Mittels vorzugsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums. Auch der Zusatz einer organischen Base wie Triethylamin, Dimethylanilin, Pyridin oder Chinolin kann günstig sein. Die Reaktionszeit liegt je nach den angewendeten Bedingungen zwischen einigen Minuten und 14 Tagen, die Reaktionstemperatur zwischen etwa 0° und 150°, normalerweise zwischen 20° und 130°.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan;'Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Verbindungen der Formel I lassen sich weiterhin bevorzugt durch Umsetzung von Verbindungen der Formel IV mit Verbindungen der Formel V erhalten. Als Verbindungen der Formel V eignen sich bevorzugt Dialkylcarbonate wie Dimethyl-, Ditrichlormethyl- oder Diethylcarbonat, Chlorameisensäureester wie Chlorameisensäuremethyl- oder -ethylester, N,N'-Carbonyldiimidazol oder Phosgen.

Die Ausgangsstoffe der Formeln IV und V sind teilweise bekannt. Sofern sie nicht bekannt sind, können sie nach an sich bekannten Methoden hergestellt werden. Die Umsetzung erfolgt in Lösungsmitteln und bei Temperaturen wie oben beschrieben.

Verbindungen der Formel VI lassen sich weiterhin reduktiv in Verbindungen der Formel I überführen. Vorzugsweise bedient man sich hierzu der katalytischen Hydrierung mit z.B. Palladium auf Aktivkohle und Wasserstoff.

Die Ausgangsstoffe der Formel VI sind teilweise bekannt. Sofern sie nicht bekannt sind, können sie nach an sich bekannten Methoden hergestellt werden. Die Reduktion erfolgt in Lösungsmitteln und bei Temperaturen wie oben beschrieben.

Es ist ferner möglich, eine Verbindung der Formel I, worin R¹ OH bedeutet, durch Alkylierung in eine Etherverbindung der Formel I umzuwandeln, in der R¹ Alkoxy bedeutet.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, femer organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und Disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Erfindungsgemäße Verbindungen der Formel I können aufgrund ihrer Molekülstruktur chiral sein und können dementsprechend in zwei enantiomeren Formen auftreten. Sie können daher in racemischer oder in optisch aktiver Form vorliegen.

Da sich die pharmazeutische Wirksamkeit der Racemate bzw. der Stereoisomeren der erfindungsgemäßen Verbindungen unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle racemischer Amine werden aus dem Gemisch durch Umsetzung mit einem optisch aktiven Trennmittel Diastereomere gebildet. Als Trennmittel eignen sich z.B. optisch aktiven Säuren, wie die R- und S-Formen von Weinsäure, Diacetylweinsäure, Dibenzoylweinsäure, Mandelsäure, Äpfelsäure, Milchsäure, geeignet N-geschützte Aminosäuren (z.B. N-Benzoylprolin oder N-Benzolsulfonylprolin) oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kieselgel fixierte chiral derivatisierte Methacrylatpolymere). Als Laufmittel eignen sich hierfür wäßrige oder alkoholische Lösungsmittelgemische wie z.B. Hexan/Isopropanol/Acetonitril z.B. im Verhältnis 82:15:3.

Salze mit physiologisch nicht unbedenklichen Säuren, z. B. Pikrate, können zur Isolierung und/oder Aufreinigung der Verbindungen der Formel I verwendet werden.

Gegenstand der Erfindung ist ferner die Verwendung der Verbindungen der Formel I und/oder ihrer physiologisch unbedenklichen Salze zur Herstellung pharmazeutischer Zubereitungen, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/ oder Netzmittel, Emulgatoren, Salze zur Beeinflussung des osmotischen Druckes, Puffersubstanzen, Farb-, Geschmacks- und /oder mehrere weitere Wirkstoffe enthalten, z.B. ein oder mehrere Vitamine.

Die erfindungsgemäßen Verbindungen gemäß Formel I werden in der Regel in Analogie zu anderen bekannten, für die beanspruchten Indikationen im Handel erhältlichen Präparaten verabreicht (z. B. Imipramin, Fluoxetin, Clomipramin), vorzugsweise in Dosierungen zwischen 0,1 mg und 500 mg, insbesondere zwischen 5 und 300 mg pro Dosierungseinheit. Die tägliche Dosierung liegt vorzugsweise zwischen etwa 0.01 und 250 mg/kg, insbesondere zwischen 0,02 und 100 mg/kg Körpergewicht.

Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel, wobei auch eine Trennung der nachfolgend beschriebenen Isomeren erfolgt, und /oder durch Kristallisation. Rf-Werte an Kieselgel; Laufmittel: Ethylacetat/Methanol 9:1.
- Massenspektrometrie:: El (Elektronenstoßionisation): M⁺
FAB (Fast Atom Bombardment): (M+H)⁺

### Beispiel 1

Eine Lösung von 5-(Methansulfonyloxymethyl)-3-p-methoxyphenyl-oxazolidin-2-on [erhältlich durch Umsetzung von 2,3-Epoxypropanol mit N-Benzyl-p-methoxyanilin zu 1-(N-Benzyl-p-methoxy-anilino-propan-2,3-diol, Hydrogenolyse zu 1-p-Methoxyanilinopropan-2,3-diol, Umsetzung mit Diethylcarbonat zu 5-Hydroxymethyl-3-p-methoxyphenyl-oxazolidin-2-on und Reaktion mit CH₃SO₂Cl] in Acetonitril wird mit äquimolaren Mengen 4-(3-Indolyl)-piperidin ("A"), Kaliumiodid und Kaliumcarbonat versetzt, 16 Stunden unter Rückfluß erhitzt und danach wie üblich aufgearbeitet. Man erhält

3-(4-Methoxyphenyl)-5-[4-(3-indolyl)-1-piperidinylmethyl]-oxazolidin-2-on, F. 151-153°.

Analog erhält man durch Umsetzung von "A"
mit (5S)-5-(Methansulfonyloxymethyl)-3-p-methoxyphenyl-oxazolidin-2-on
   (5S)-(-)3-(4-Methoxyphenyl)-5-[4-(3-indolyl)-1-piperidinylmethyl]-oxazolidin-2-on, Hydrochlorid, F. 234-236°, α_{D}²⁰ -56° (c=1, Methanol);
mit (5S)-5-(Methansulfonyloxymethyl)-3-p-chlorphenyl-oxazolidin-2-on
   (5S)-(-)3-(4-Chlorphenyl)-5-[4-(3-indolyl)-1-piperidinylmethyl]-oxazolidin-2-on, F. 188-189°, α_{D}²⁰ -28° (c=1, DMSO); Hydrochlorid, F. 260-263°;
mit 5-(Methansulfonyloxymethyl)-3-p-cyanphenyl-oxazolidin-2-on
   3-(4-Cyanphenyl)-5-[4-(3-indolyl)-1-piperidinylmethyl]-oxazolidin-2-on;
mit 5-(Methansulfonyloxymethyl)-3-phenyl-oxazolidin-2-on
   3-Phenyl-5-[4-(3-indolyl)-1-piperidinylmethyl]-oxazolidin-2-on;
mit 5-(Methansulfonyloxymethyl)-3-p-fluorphenyl-oxazolidin-2-on
   3-(4-Fluorphenyl)-5-[4-(3-indolyl)-1-piperidinylmethyl]-oxazolidin-2-on.

Analog erhält man durch Umsetzung von 4-(5H-1,3-dioxolo[4,5-f]-indol-7-yl)-piperidin ("B")
mit (5S)-5-(Methansulfonyloxymethyl)-3-p-methoxyphenyl-oxazolidin-2-on
   (5S)-(-)3-(4-Methoxyphenyl)-5-[4-(5H-1,3-dioxolo[4,5-f]-indol-7-yl)-1-piperidinylmethyl]-oxazolidin-2-on, Hydrochlorid, F. 232-234°, α_{D}²⁰ -50,5° (c=1, Methanol);
mit (5S)-5-(Methansulfonyloxymethyl)-3-p-chlorphenyl-oxazolidin-2-on
   (5S)-(-)3-(4-Chlorphenyl)-5-[4-(5H-1,3-dioxolo[4,5-f]-indol-7-yl)-1-piperidinylmethyl]-oxazolidin-2-on.

Analog erhält man durch Umsetzung von 4-(5-Fluor-3-indolyl)-piperidin ("C")
mit (5S)-5-(Methansulfonyloxymethyl)-3-p-methoxyphenyl-oxazolidin-2-on
   (5S)-(-)3-(4-Methoxyphenyl)-5-[4-(5-fluor-3-indolyl)-1-piperidinylmethyl]-oxazolidin-2-on, Hydrochlorid, F. 233-234°, α_{D}²⁰ -58,5° (c=1, DMSO);
mit 5-(Methansulfonyloxymethyl)-3-p-cyanphenyl-oxazolidin-2-on
   3-(4-Cyanphenyl)-5-[4-(5-fluor-3-indolyl)-1-piperidinylmethyl]-oxazolidin-2-on, Hydrochlorid, F. 290-292°;
mit (5S)-5-(Methansulfonyloxymethyl)-3-p-cyanphenyl-oxazolidin-2-on
   (5S)-(-)3-(4-Cyanphenyl)-5-[4-(5-fluor-3-indolyl)-1-piperidinylmethyl]-oxazolidin-2-on, Hydrochlorid, F. 203-204°, α_{D}²⁰ -36,5° (c=1, DMSO);
mit (5R)-5-(Methansulfonyloxymethyl)-3-p-cyanphenyl-oxazolidin-2-on
   (5R)-(+)3-(4-Cyanphenyl)-5-[4-(5-fluor-3-indolyl)-1-piperidinylmethyl]-oxazolidin-2-on, Hydrochlorid, F. 286-287°, α_{D}²⁰ +41,5° (c=1, DMSO).

Analog erhält man durch Umsetzung von 4-(5-Cyan-3-indolyl)-piperidin ("D")
mit 5-(Methansulfonyloxymethyl)-3-p-cyanphenyl-oxazolidin-2-on
   3-(4-Cyanphenyl)-5-[4-(5-cyan-3-indolyl)-1-piperidinylmethyl]-oxazolidin-2-on, Hydrochlorid, F. 290°;
mit (5S)-5-(Methansulfonyloxymethyl)-3-p-fluorphenyl-oxazolidin-2-on
   (5S)-(-)-3-(4-Fluorphenyl)-5-[4-(5-cyan-3-indolyl)-1-piperidinylmethyl]-oxazolidin-2-on, Hydrochlorid, F. 252-253°;
mit (5S)-5-(Methansulfonyloxymethyl)-3-p-cyanphenyl-oxazolidin-2-on
   (5S)-(-)-3-(4-Cyanphenyl)-5-[4-(5-cyan-3-indolyl)-1-piperidinylmethyl]-oxazolidin-2-on, Hydrochlorid, F. 270-271°, α_{D}²⁰ -38,7° (c=1, DMSO);
mit (5R)-5-(Methansulfonyloxymethyl)-3-p-cyanphenyl-oxazolidin-2-on
   (5R)-(+)-3-(4-Cyanphenyl)-5-[4-(5-cyan-3-indolyl)-1 -piperidinylmethyl]-oxazolidin-2-on, Hydrochlorid, F. 270-272°, α_{D}²⁰ +37,7° (c=1, DMSO);
mit 5-(Methansulfonyloxymethyl)-3-p-fluorphenyl-oxazolidin-2-on
   3-(4-Fluorphenyl)-5-[4-(5-cyan-3-indolyl)-1-piperidinylmethyl]-oxazolidin-2-on, Hydrochlorid, F. 264-268°;
mit 5-(Methansulfonyloxymethyl)-3-phenyl-oxazolidin-2-on
   3-Phenyl-5-[4-(5-cyan-3-indolyl)-1-piperidinylmethyl]-oxazolidin-2-on, Hydrochlorid Hydrat, F. 183-185°;
mit (5R)-5-(Methansulfonyloxymethyl)-3-p-fluorphenyl-oxazolidin-2-on
   (5R)-(+)-3-(4-Fluorphenyl)-5-[4-(5-cyan-3-indolyl)-1-piperidinylmethyl]-oxazolidin-2-on, Hydrochlorid, F. 184-188°, α_{D}²⁰ +27,2° (c=1, DMSO),

Analog erhält man durch Umsetzung von 4-(6-Fluor-3-indolyl)-piperidin ("E")
mit (5S)-5-(Methansulfonyloxymethyl)-3-p-cyanphenyl-oxazolidin-2-on
   (5S)-(-)-3-(4-Cyanphenyl)-5-[4-(6-fluor-3-indolyl)-1-piperidinylmethyl]-oxazolidin-2-on, Hydrochlorid, F. 287-288°, α_{D}²⁰ -38,4° (c=1, DMSO);
mit 5-(Methansulfonyloxymethyl)-3-p-fluorphenyl-oxazolidin-2-on
   3-(4-Fluorphenyl)-5-[4-(6-fluor-3-indolyl)-1-piperidinylmethyl]-oxazolidin-2-on, Hydrochlorid, F. 257-259°;
mit (5R)-5-(Methansulfonyloxymethyl)-3-p-cyanphenyl-oxazolidin-2-on
   (5R)-(+)-3-(4-Cyanphenyl)-5-[4-(6-fluor-3-indolyl)-1-piperidinylmethyl]-oxazolidin-2-on, Hydrochlorid, F. 288-290°, α_{D}²⁰ +38,8° (c=1, DMSO);
mit 5-(Methansulfonyloxymethyl)-3-phenyl-oxazolidin-2-on
   3-Phenyl-5-[4-(6-fluor-3-indolyl)-1-piperidinylmethyl]-oxazolidin-2-on, Hydrochlorid, F. 234-236°.

### Beispiel 2

Eine Lösung von 1-[4-(3-lndolyl)-piperidin-1-yl]-3-(4-methoxy-anilino)-propan-2-ol in Dichlormethan wird mit äquimolaren Mengen Ditrichlormethyl-carbonat versetzt und 5 Stunden gerührt. Nach üblicher Aufarbeitung erhält man 3-(4-Methoxyphenyl)-5-[4-(3-indolyl)-1-piperidinylmethyl]-oxazolidin-2-on, F. 151-153°.

### Beispiel 3

Durch eine Lösung von 5-[4-(3-indolyl)-3,6-dihydro-2H-pyridin-1-ylmethyl]-3-(4-methoxyphenyl)-oxazolidin-2-on [erhältlich durch Dehydratisierung von 3-(4-Methoxyphenyl)-5-[4-(3-indolyl)-4-hydroxy-1-piperidinylmethyl]-oxazolidin-2-on, welches durch Reaktion von 3-(4-Methoxyphenyl)-5-[4-oxo-1-piperidinylmethyl]-oxazolidin-2-on mit Indol hergestellt wurde] in Methanol leitet man in Gegenwart von Palladium (10% auf Aktivkohle) 1 Stunde Wasserstoff. Nach Abtrennung des Katalysators und üblicher Aufarbeitung erhält man 3-(4-Methoxyphenyl)-5-[4-(3-indolyl)-1-piperidinylmethyl]-oxazolidin-2-on, F. 151-153°.

Die nachfolgenden Beispiele betreffen pharmazeutische Zubereitungen:

### Beispiel A: Injektionsgläser

Eine Lösung von 100 g eines Wirkstoffs der Formel I und 5 g Dinatriumhydrogenphosphat wird in 3 I zweifach destilliertem Wasser mit 2 n Salzsäure auf pH 6.5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffs der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffs der Formel I, 9.38 g NaH₂PO₄2H₂O, 28.48 g Na₂HPO₄·12H₂O und 0.1 g Benzalkoniumchlorid in 940 ml zweifach destilliertem Wasser. Man stellt auf pH 6.8 ein, füllt auf 1 I auf und sterilisiert durch Bestrahlung.

### Beispiel D: Salbe

Man mischt 500 mg eines Wirkstoffs der Formel I mit 99.5 g Vaseline unter aseptischen Bedingungen.

### Beispiel E: Tabletten

Ein Gemisch von 1 kg Wirkstoff der Formel I, 4 kg Laktose, 1.2 kg Kartoffelstärke, 0.2 kg Talk und 0.1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel I 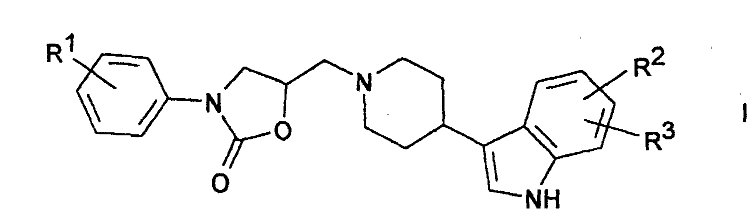 worin
R¹ H, CN, Hal oder OA,
R², R³ jeweils unabhängig voneinander H, CN, Hal oder OA,
R² und R³ zusammen auch Methylendioxy,
A H, CF₃ oder Alkyl mit 1-6 C-Atomen und
Hal F, Cl, Br, I
bedeuten,
sowie deren Salze.

2. Enantiomere der Verbindungen der Formel I gemäß Anspruch 1.

3. Verbindungen der Formel I gemäß der Ansprüche 1 oder 2
a) (5S)-(-)-3-(4-Chlorphenyl]-5-[4-(3-indolyl)-1-piperidinylmethyl]-oxazolidin-2-on;
b) 3-(4-Cyanphenyl)-5-[4-(5-cyan-3-indolyl)-1-piperidinylmethyl]-oxazolidin-2-on;
c) (5S)-(-)-3-(4-Cyanphenyl)-5-[4-(6-fluor-3-indolyl)-1-piperidinylmethyl]-oxazolidin-2-on;
d) (5R)-(+)-3-(4-Cyanphenyl)-5-[4-(5-fluor-3-indolyl)-1-piperidinylmethyl]-oxazolidin-2-on;
e) (5R)-(+)-3-(4-Cyanphenyl)-5-[4-(6-fluor-3-indolyl)-1-piperidinylmethyl]-oxazolidin-2-on;
f) 3-Phenyl-5-[4-(5-cyan-3-indolyl)-1-piperidinyl-methyl]-oxazolidin-2-on;
g) 3-Phenyl-5-[4-(6-fluor-3-indolyl)-1-piperidinyl-methyl]-oxazolidin-2-on;
sowie deren Salze.

4. Verfahren zur Herstellung von Verbindungen der Formel I gemäß Anspruch 1, **dadurch gekennzeichnet, daß** man
a) eine Verbindung der Formel II 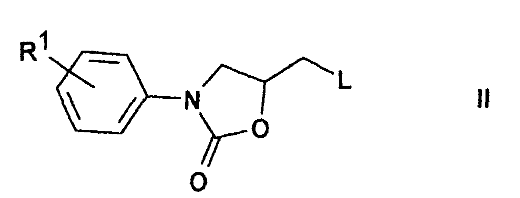 worin R¹ die in Anspruch 1 angegebene Bedeutung hat und
L Cl, Br, I oder eine freie oder reaktionsfähig funktionell abgewandelte OH-Gruppe bedeutet,
mit einer Verbindung der Formel III 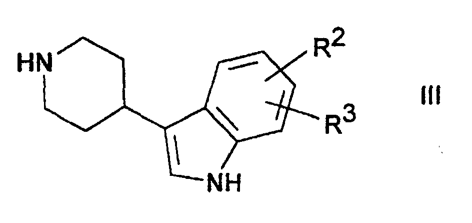 worin R² und R³ die in Anspruch 1 gegebenen Bedeutungen haben,
umsetzt,
oder
b) eine Verbindung der Formel IV 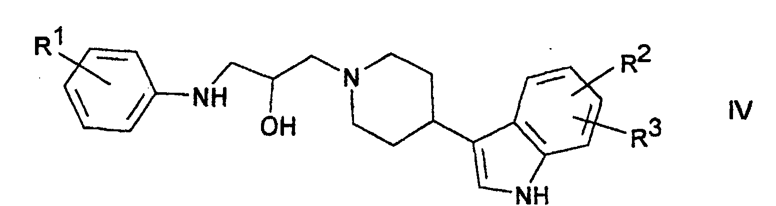 worin R¹, R² und R³ die in Anspruch 1 angegebenen Bedeutungen haben,
mit einer Verbindung der Formel V 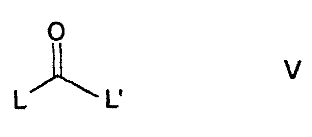 worin L und L' jeweils unabhängig voneinander Cl, Br, I oder eine freie oder reaktionsfähig funktionell abgewandelte OH-Gruppe bedeutet,
umsetzt,
oder
c) eine Verbindung der Formel VI 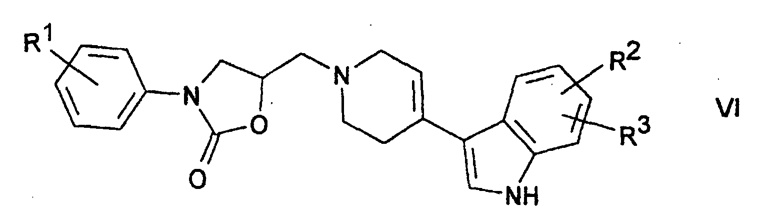
worin R¹, R² und R³ die in Anspruch 1 angegebenen Bedeutungen haben,
hydriert,
undloder daß man eine basische Verbindung der Formel I durch Behandeln mit einer Säure in eines ihrer Salze überführt.

5. Verfahren zur Herstellung pharmazeutischer Zubereitungen, **dadurch gekennzeichnet, daß** man eine Verbindung der Formel I nach Anspruch 1 und/oder eines ihrer physiologisch verträglichen Salze oder eines ihrer Enantiomere zusammen mit mindestens einem festen, flüssigen oder halbflüssigen Träger- oder Hilfsstoff und gegebenenfalls in Kombination mit einem oder mehreren weiteren Wirkstoffen in eine geeignete Dosierungsform bringt.

6. Pharmazeutische Zubereitung, **gekennzeichnet durch** einen Gehalt an mindestens einer Verbindung nach einem der Ansprüche 1 oder 2 und/oder einem ihrer physiologisch unbedenklichen Salze.

7. Verbindungen der Formel I nach Anspruch 1 und ihre physiologisch unbedenklichen Salze zur Bekämpfung der Folgen von Schlaganfall und cerebraler Ischämien, zur Behandlung von Zwangsverhalten (OCD), Angstzuständen, Panikattaken, Depressionen, Psychosen, Schizophrenie und des Morbus Parkinson.

8. Arzneimittel der Formel I nach Anspruch 1 und ihre physiologisch unbedenklichen Salze als 5-HT_{2A}-Antagonisten mit 5-HT-reuptake-hemmender Wirkung.

9. Verwendung von Verbindungen der Formel I nach Anspruch 1 und/oder ihre physiologisch unbedenklichen Salze zur Herstellung eines Arzneimittels.

10. Verwendung von Verbindungen der Formel I nach Anspruch 1 und/oder ihrer physiologisch unbedenklichen Salze zur Herstellung eines Arzneimittels zur Bekämpfung der Folgen von Schlaganfall und cerebraler Ischämien, zur Behandlung von Zwangsverhalten (OCD), Angstzuständen, Panikattacken, Depression, Psychosen, Schizophrenie und des Morbus Parkinson.

## Claims

1. Compounds of the formula I 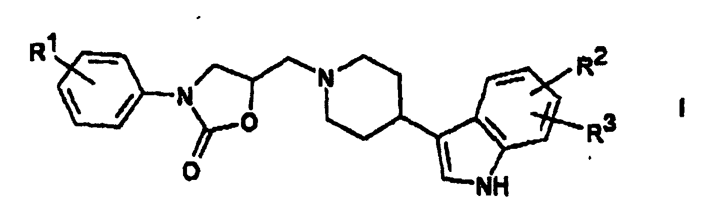 in which
R¹ is H, CN, Hal or OA,
R² and R³ are each, independently of one another, H, CN, Hal or OA,
R² and R³ together are alternatively methylenedioxy,
A is H, CF₃ or alkyl having 1-6 carbon atoms, and
Hal is F, Cl, Br or I,
and salts thereof.

2. Enantiomers of the compounds of the formula I according to Claim 1.

3. Compounds of the formula I according to Claims 1 or 2
a) (5S)-(-)-3-(4-chlorophenyl)-5-[4-(3-indolyl)-1-piperidinylmethyl]-oxazolidin-2-one;
b) 3-(4-cyanophenyl)-5-[4-(5-cyano-3-indolyl)-1-piperidinylmethyl]-oxazolidin-2-one;
c) (5S)-(-)-3-(4-cyanophenyl)-5-[4-(6-fluoro-3-indolyl)-1-piperidinylmethyl]oxazolidin-2-one;
d) (5R)-(+)-3-(4-cyanophenyl)-5-[4-(5-fluoro-3-indolyl)-1-piperidinylmethyl]oxazolidin-2-one;
e) (5R)-(+)-3-(4-cyanophenyl)-5-[4-(6-fluoro-3-indolyl)-1-piperidinylmethyl]oxazolidin-2-one;
f) 3-phenyl-5-[4-(5-cyano-3-indolyl)-1-piperidinylmethyl]oxazolidin-2-one;
g) 3-phenyl-5-[4-(6-fluoro-3-indolyl)-1-piperidinylmethyl]oxazolidin-2-one;
and salts thereof.

4. Process for the preparation of compounds of the formula I according to Claim 1, **characterised in that**
a) a compound of the formula II 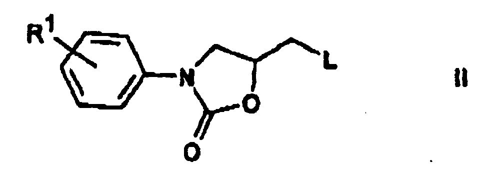 in which R¹ is as defined in Claim 1, and
L is Cl, Br, I or a free or reactively functionally modified OH group,
is reacted with a compound of the formula III 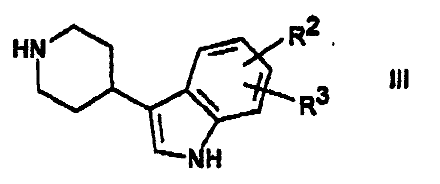 in which R² and R³ are as defined in Claim 1,
or
b) a compound of the formula IV 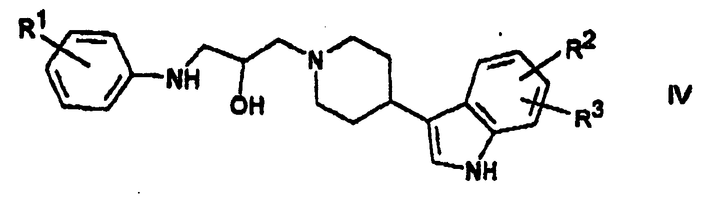 in which R¹, R² and R³ are as defined in Claim 1,
is reacted with a compound of the formula V 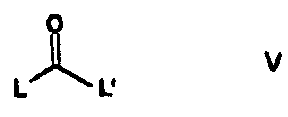 in which L and L' are each, independently of one another, Cl, Br, I or a free or reactively functionally modified OH group,
or
c) a compound of the formula VI 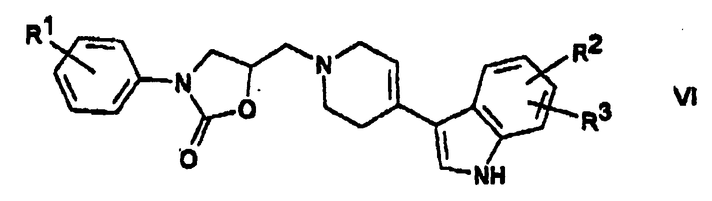 in which R¹, R² and R³ are as defined in Claim 1,
is hydrogenated,
and/or **in that** a basic compound of the formula I is converted into one of its salts by treatment with an acid.

5. Process for the preparation of pharmaceutical preparations, **characterised in that** a compound of the formula I according to Claim 1 and/or one of its physiologically acceptable salts or one of its enantiomers is converted into a suitable dosage form together with at least one solid, liquid or semi-liquid excipient or adjuvant and, if desired, in combination with one or more further active ingredients.

6. Pharmaceutical preparation, **characterised by** a content of at least one compound of the formula I according to one of Claims 1 or 2 and/or one of its physiologically acceptable salts.

7. Compounds of the formula I according to Claim 1 and physiologically acceptable salts thereof for combating the consequences of strokes and cerebral ischaemia, for the treatment of obsessive-compulsive disorder (OCD), anxiety states, panic attacks, depression, pyschoses, schizophrenia and Parkinson's disease.

8. Medicaments of the formula I according to Claim 1 and physiologically acceptable salts thereof as 5-HT_{2A} antagonists having a 5-HT reuptake-inhibiting action.

9. Use of compounds of the formula I according to Claim 1 and/or physiologically acceptable salts thereof for the preparation of a medicament.

10. Use of compounds of the formula I according to Claim 1 and/or physiologically acceptable salts thereof for the preparation of a medicament for combating the consequences of strokes and cerebral ischaemia, for the treatment of obsessive-compulsive disorder (OCD), anxiety states, panic attacks, depression, pyschoses, schizophrenia and Parkinson's disease.

## Revendications

1. Composés de formule I 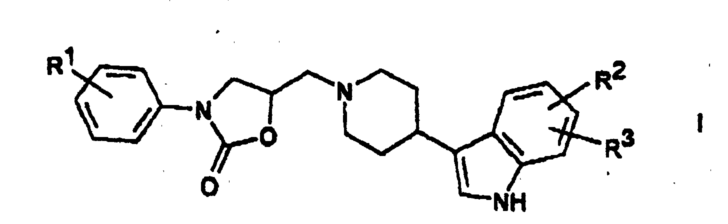 dans laquelle
R¹ représente H, CN, Hal ou OA,
R² et R³ représentent chacun, indépendamment l'un de l'autre, H, CN, Hal ou OA,
R² et R³ représentent ensemble de manière alternative méthylènedioxy,
A représente H, CF₃ ou alkyle ayant de 1 à 6 atomes de carbone, et
Hal représente F, Cl, Br ou I,
et les sels de ceux-ci.

2. Enantiomères des composés de formule I selon la revendication 1.

3. Composés de formule I selon la revendication 1 ou 2
a) la (5S)-(-)-3-(4-chlorophényl)-5-[4-(3-indolyl)-1-pipéridinylméthyl]-oxazolidin-2-one;
b) la 3-(4-cyanophényl)-5-[4-(5-cyano-3-indolyl)-1-pipéridinylméthyl]-oxazolidin-2-one;
c) la (5S)-(-)-3-(4-cyanophényl)-5-[4-(6-fluoro-3-indolyl)-1-pipéridinylméthyl]oxazolidin-2-one;
d) la (5R)-(+)-3-(4-cyanophényl)-5-[4-(5-fluoro-3-indolyl)-1-pipéridinylméthyl]oxazolidin-2-one;
e) la (5R)-(+)-3-(4-cyanophényl)-5-[4-(6-fluoro-3-indolyl)-1-pipéridinylméthyl]oxazolidin-2-one;
f) la 3-phényl-5-[4-(5-cyano-3-indolyl)-1-pipéridinylméthyl]oxazolidin-2-one;
g) la 3-phényl-5-[4-(6-fluoro-3-indolyl)-1-pipéridinylméthyl]oxazolidin-2-one;
et les sels de ceux-ci.

4. Procédé de préparation de composés de formule I selon la revendication 1, **caractérisé en ce que**
a) un composé de formule II 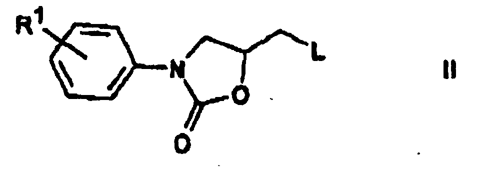 dans laquelle R¹ est tel que défini selon la revendication 1, et
L représente Cl, Br, I ou un groupement OH libre ou modifié de manière réactive et fonctionnelle,
est réagi avec un composé de formule III 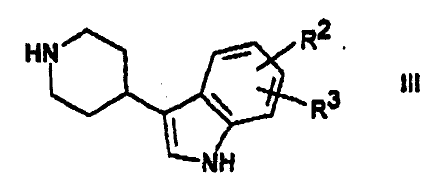 dans laquelle R² et R³ sont tels que définis selon la revendication 1,
ou
b) un composé de formule IV 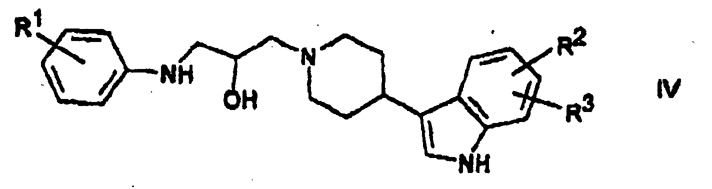 dans laquelle R¹, R² et R³ sont tels que définis selon la revendication 1,
est réagi avec un composé de formule V 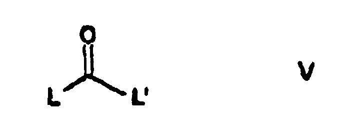 dans laquelle L et L' représentent chacun, indépendamment l'un de l'autre, Cl, Br, I ou un groupement OH libre ou modifié de manière réactive et fonctionnelle,
ou
c) un composé de formule VI 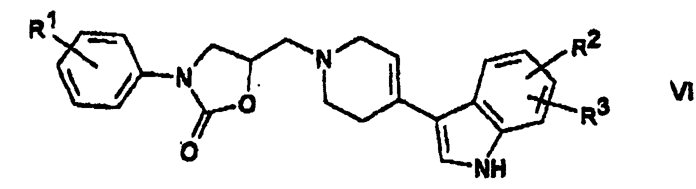
dans laquelle R¹, R² et R³ sont tels que définis selon la revendication 1,
est hydrogéné,
et/ou **en ce qu'**un composé basique de formule I est converti en l'un de ses sels par un traitement par un acide.

5. Procédé de préparation de préparations pharmaceutiques, **caractérisé en ce qu'**un composé de formule I selon la revendication 1 et/ou l'un de ses sels physiologiquement acceptables ou l'un de ses énantiomères est mis sous une forme de dosage convenable conjointement avec au moins un excipient ou adjuvant solide, liquide ou semi-liquide et, si on le désire, en association avec un ou plusieurs ingrédients actifs supplémentaires.

6. Préparation pharmaceutique, **caractérisée par** une teneur en au moins un composé de formule I selon l'une des revendications 1 et 2 et/ou l'un de ses sels physiologiquement acceptables.

7. Composés de formule I selon la revendication 1 et les sels physiologiquement acceptables de ceux-ci pour lutter contre les conséquences d'accidents vasculaires cérébraux et de l'ischémie cérébrale, pour le traitement du trouble obsessionnel-compulsif (TOC), des états d'anxiété, des attaques de panique, de la dépression, des psychoses, de la schizophrénie et de la maladie de Parkinson.

8. Médicaments de formule I selon la revendication 1 et les sels physiologiquement acceptables de ceux-ci comme antagonistes de 5-HT_{2A} ayant une action d'inhibition de recaptage de 5-HT.

9. Utilisation de composés de formule I selon la revendication 1 et/ou de sels physiologiquement acceptables de ceux-ci pour la préparation d'un médicament.

10. Utilisation de composés de formule I selon la revendication 1 et/ou de sels physiologiquement acceptables de ceux-ci pour la préparation d'un médicament pour lutter contre les conséquences d'accidents vasculaires cérébraux et de l'ischémie cérébrale, pour le traitement du trouble obsessionnel-compulsif (TOC), des états d'anxiété, des attaques de panique, de la dépression, des psychoses, de la schizophrénie et de la maladie de Parkinson.
